# EUROPEAN PATENT SPECIFICATION

(11) **EP 2 259 800 B1**
(45) Date of publication and mention of the grant of the patent: **07.05.2014**
(21) Application number: 09716778.7
(22) Date of filing: 04.03.2009
(51) Int. Cl.: A61K 45/06, A61P 35/00, A61K 31/5377

(54) **Use of 5-(2,6-di-morpholin-4-yl-pyrimidin-4-yl)-4-trifluoromethyl-pyridin-2-ylamine for the treatment of non small cell lung carcinoma with acquired resistance to epidermal growth factor receptor (EGFR) modulators.**
Verwendung von 5-(2,6-Di-Morpholin-4-yl-Pyrimidin-4-yl)-4-Trifluoromethyl-Pyridin-2-ylamin zur Behandlung von nicht-kleinzelligem Bronchialkarzinom mit erworbener Resistenz gegenüber Modulatoren des EGF (Epidermal Growth Factor)-Rezeptors
Utilisation de 5-(2,6-di-morpholin-4-yl-pyrimidin-4-yl)-4-trifluoromethyl-pyridin-2-ylamine pour le traitement de carcinome du poumon non à petites cellule ayant acquis une résistance aux modulateurs du recepteur du facteur de croissance epidermique (EGFR)

(30) Priority: 05.03.2008 EP 08152326
(43) Date of publication of application: 15.12.2010
(60) Divisional application: 13154400.9
(73) Proprietor: Novartis AG, 4056 Basel (CH)
(72) Inventor: GARCIA-ECHEVERRIA, Carlos, 4052 Basel (CH); MAIRA, Sauveur-Michel, 68440 Habsheim (FR)
(74) Representative: Gruber, Markus
(86) International application number: PCT/EP2009/052564
(87) International publication number: WO 2009/109605

(56) References cited:
- WO-A-2004/048365
- WO-A-2007/084786
- PAO W ET AL: "Acquired resistance of lung adenocarcinomas to gefitinib or erlotinib is associated with a second mutation in the EGFR kinase domain", PLOS MEDICINE, PUBLIC LIBRARY OF SCIENCE, US, vol. 2, no. 3, 1 March 2005 (2005-03-01), pages 225-235, XP002359961, ISSN: 1549-1676, DOI: 10.1371/JOURNAL.PMED.0020225

## Description

The present invention relates to the use of a specific pyrimidine derivative in the treatment of Epidermal Growth Factor Receptor (EGFR) family members (including EGFR1 also known as HER1 or Erb-B1 EGFR2 also known as HER2 or Erb-B2: and EGFR3 also known as HER3 or Erb-B3) dependent diseases or diseases that have acquired resistance to some agents that target EGFR family members, use of said compounds for the manufacture of pharmaceutical compositions for the treatment of said diseases. Combinations of said compounds with EGFR modulators for said use, methods of treating said diseases with said compounds, and pharmaceutical preparations for the treatment of said diseases comprising said compounds, alone or in combination, especially with an EGFR modulator are also described.

Somatic mutations in the tyrosine kinase domain of EGFR has been associated with the clinical response to EGFR tyrosine kinase inhibitor such as Gefitinib (Iressa®) or Erlotinib (Tarceva®) (Paez et al., EGFR mutations in lung cancer: correlation with clinical response to gefitinib therapy, science, vol 304, 1497-1500). Acquired resistance to EGFR modulators occurs in patients who initially responded clinically to therapy, but then developed progressive tumors. Refractory response to EGFR kinase inhibitors is exemplified with the secondary resistant mutation T790M (Kobayashi et al.; EGFR mutation and resistance of non-small cell lung cancer to gefitinib, N. Ehgl J Med, Vol 352, 786-792), which is comparable to the resistance mutation(s) observed for Gleevec/Glivec or Dasatinib in chronic myelogenous leukemia (CML) (Garre et al.; Bcr-Abl point mutants isolated from patients with imatinib mesylate resistant chronic leukemia reamin sensitive to inhibitors of the Bcr-Abl chaperone heat shock protein 90, Blood, vol 100, 3041-3044*)* or GIST patients (Antonescu et al.; Acquired resistance to Imatinib in gastrointestinal stromal tumors occurs through secondary gene mutation, Clin Cancer Res, Vol 11, 4182-4190)*.*

Evidence that activation of the PI3K pathway downstream of activated EGFR exists in the literature. Thus, genetic ablation of the PI3K catalytic subunit (p110) in mouse embryo fibroblast renders cells resistant for transformation by an activated form of EGFR *(*Zhao et al.; The p110 alpha isoform of PI3K is essential for proper growth factor signaling and oncogenic transformation, PNAS, vol 103, 16296-16300). HER3 (ErbB-3), one of the four member of the EGFR family and partner of HER1 (EGFR1) is often overexpressed in EGFR inhibitors sensitive tumors, and that is correlated with constitutive PI3K recruitment and activation *(*Engelman et al.; ErbB-3 mediates phosphoinositide 3-kinase activity in gefitinib-sensitive non small cell lung cancer cell lines, PNAS vol 102, 3788-3793*; Sergina et al.;* Escape *from HER-family tyrosine kinase inhibitor therapy by the kinase-inactive HER* 3). The genetic and biochemical characterization of tumor biopsies and tumor cell lines harboring EGFR amplification and EGFR inhibitor resistance have revealed a constitutive activation status of the PI3K pathway (Engelman et al.; Allelic disruption obscures detection of a biologically significant resistance mutation in EGFR amplified lung cancer, The Journal of Clinical Investigation, vol 116, 2695-2706)*.*

WO 2004/084 365 describes 2,4,6-trisubstituted pyrimidines as PI3K inhibitors.

Surprisingly, it has been found that specific pyrimidine derivatives, which have been described in WO07/084786 provoke strong anti-proliferative activity and an in vivo antitumor response of breast and lung cancer cell lines with amplified EGFRs and/or mutated EGFR1 as single agent and in combination with EGFR kinase modulators. Therefore, said compounds are useful for the treatment of EGFR dependent disease.

Specific pyrimidine derivatives which are suitable for the present disclosure, their preparation and suitable pharmaceutical formulations containing the same are described in WO07/084786 and include compounds of formula I 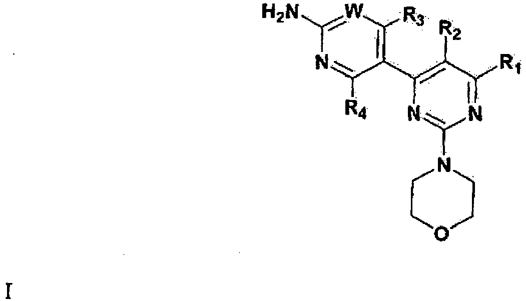 or a stereoisomer, tautomer, or pharmaceutically acceptable salt thereof, wherein, W is CR_{w} or N, wherein R_{w} is selected from the group consisting of
(1) hydrogen,
(2) cyano,
(3) halogen,
(4) methyl,
(5) trifluoromethyl,
(6) sulfonamido;
R₁ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR_{1a,}
(13) -CO₂R₁ₐ,
(14) -CONR₁ₐR_{1b},
(15) -NR₁ₐR_{1b},
(16) -NR₁ₐCOR_{1b},
(17) -NR₁ₐSO₂R_{1b},
(18) -OCOR₁ₐ,
(19) -OR₁ₐ,
(20) -SR₁ₐ,
(21) -SOR₁ₐ,
(22) -SO₂R₁ₐ, and
(23) -SO₂NR₁ₐR_{1b},
wherein R₁ₐ, and R_{1b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl;
R₂ is selected from the group consisting
(1) hydrogen,
(2) cyano,
(3) nitro,
(4) halogen,
(5) hydroxy,
(6) amino,
(7) substituted and unsubstituted alkyl,
(8) -COR₂ₐ, and
(9) -NR₂ₐCOR_{2b},
wherein R₂ₐ, and R_{2b} are independently selected from the group consisting of
(a) hydrogen, and
(b) substituted or unsubstituted alkyl;
R₃ is selected from the group consisting of
(1) hydrogen,
(2) cyano,
(3) nitro,
(4) halogen,
(5) substituted and unsubstituted alkyl,
(6) substituted and unsubstituted alkenyl,
(7) substituted and unsubstituted alkynyl,
(8) substituted and unsubstituted aryl,
(9) substituted and unsubstituted heteroaryl,
(10) substituted and unsubstituted heterocyclyl,
(11) substituted and unsubstituted cycloalkyl,
(12) -COR₃ₐ,
(13) -NR₃ₐR_{3b},
(14) -NR₃ₐCOR_{3b},
(15) -NR₃ₐSO₂R_{3b},
(16) -OR₃ₐ,
(17) -SR₃ₐ,
(18) -SOR₃ₐ,
(19) -SO₂R₃ₐ, and
(20) -SO₂NR₃ₐR_{3b},
wherein R₃ₐ, and R_{3b} are independently selected from the group consisting of
(a) hydrogen,
(b) substituted or unsubstituted alkyl,
(c) substituted and unsubstituted aryl,
(d) substituted and unsubstituted heteroaryl,
(e) substituted and unsubstituted heterocyclyl, and
(f) substituted and unsubstituted cycloalkyl; and
R₄ is selected from the group consisting of
(1) hydrogen, and
(2) halogen.
The radicals and symbols as used in the definition of a compound of formula I have the meanings as disclosed in WO07/084786.

A compound useful in the present invention is 5-(2,6-di-morpholin-4-yl-pyrimidin-4-yl)-4-trifluoromethyl-pyridin-2-ylamine (Compound A). The synthesis of 5-(2,6-di-morpholin-4-yl-pyrimidin-4-yl)-4-trifluoromethyl-pyridin-2-ylamine is described in WO07/084786 as Example 10.

Compounds that target members of the EGFR family according to the present disclosure include EGFR family kinase modulators, compounds that alter EGFR expression levels or elicit a cellular immune reponse linked to the expression of EGFR family members in the tumor cells. Preferrable EGFR modulators exhibit their activity as inhibitors of EGFR functional activity Compounds that target members of the EGFR family according to the present disclosure include without limitation gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922.

### Short description of the figures

Figure 1 shows the antitumor activity of Compound A against the EGFR inhibitor resistant NSCLC cell line NCI-H1975.
   Female Harlan athymic mice (n = 6), bearing s.c NCI-H1975 tumors are treated p.o. with the PI3K inhibitor Compound A at 50mg/kg every day for the duration of the treatment. * p<0.05 (Dunnet's vs controls).
Figure 2 shows the mean body weight of vehicle and Compound A treated groups during the efficacy in vivo study performed with mice bearing the s.c. the EGFR inhibitor resistant NSCLC cell line NCI-H1975.

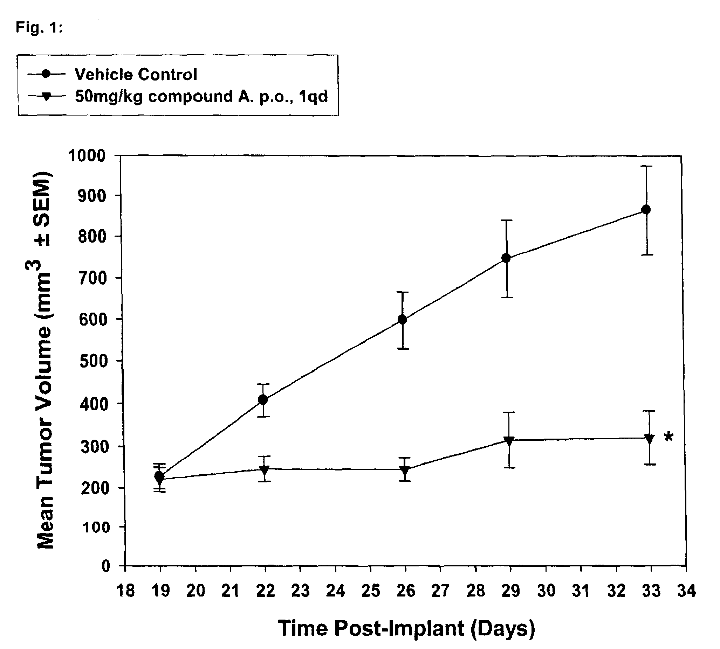

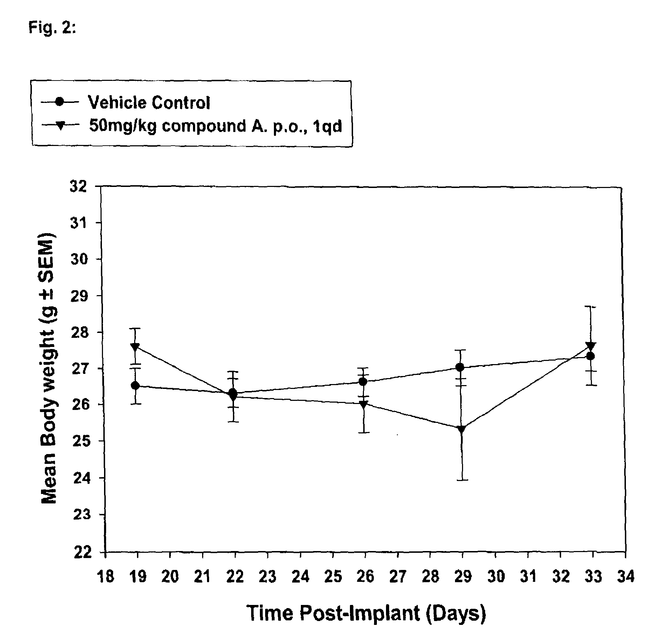

Non small cell lung carcinoma cell lines with EGFR mutant forms, but refractory to EGFR inhibition therapy, are valuable models to test the sensitivity of PI3K inhibitors like the compounds of formula I in such genetic background. The NCI-H1975 cell line is such a model as is highly tumorigenic in vivo. Moreover, it contains HER1 bearing the T790M gatekeeper mutation that renders the kinase resistant to catalytic inhibition by compounds such as gefitinib. The *in vivo* antitumor activity of PI3K inhibitors like the compounds of formula I is tested against this EGFR driven and EGFR tyrosine kinase inhibitor resistant tumor model (Figure 1). Surprisingly administration of Compound A causes *in vivo* tumor growth inhibition. Compound A is well tolerated - no statistically significant difference in body weight between the control and treatment groups can be observed (Figure 2).

A compound of formula I, especially Compound A, is therefore useful for the treatment of such EGFR dependent diseases, especially malignancies, or EGFR family members acquired resistance driven diseases. Diseases or malignancies with an established or potential molecular link to dysregulation of EGFR activity are, for instance, described in *"*Mendelsohn and Baselga; Status of Epidermal Growth Factor Receptor Antagonists in the Biology and Treatment of Cancer, Journal of Clinical Oncology, 2787-2799*"; "*Mendelsohn and Baselga; Epidermal Growth Factor Receptor Targeting in Cancer, Seminars in Oncology, Vol 33, 369-385*";* Irmer et al., EGFR kinase domain mutations - functional impact and relevance for lung cancer therapy, Oncogene, 1-9*;* Roche-Lima et al., EGFR targeting of solid tumors; Cancer Control, 2007, Vol 14 (3), 295-304).

According to the present invention the treatment of the following EGFR dependent diseases, especially malignancies, with Compound A, or a pharmaceutically acceptable salt thereof, is non small cell lung carcinoma.

The present disclosure relates to the use of acompound of formula I as described above, or a tautomer thereof, or a pharmaceutically acceptable salt, or a hydrate or solvate thereof for the manufacture of a pharmaceutical preparation for the treatment of an EGFR dependent disease.

Furthermore, the present invention relates to the use of 5-(2,6-di-morpholin-4-yl-pyrimidin-4-yl)-4-trifluoromethyl-pyridin-2-ylamine, or a pharmaceutically acceptable salt thereof, for the manufacture of a pharmaceutical preparation for the treatment of an EGFR dependent disease, or malignancy, wherein said disease or malignancy is resistant to the treatment with an EGFR modulator which is selected from the group consisting of gefitinib, erlotinib, lapatinib, cetuximab, nimotuzumab, panitumumab and trastuzumab, and wherein the EGFR dependent disease is non small cell lung carcinoma.

The resistance to the treatment with an EGFR modulator may have been acquired during treatment with said EGFR modulator. It is also disclosed that such resistance can be due to a mutation or mutations in the protein.

In particular, the present disclosure describes the treatment of a disease or malignancy that is dependent on EGFR family members or has aquired resistance during treatment with an EGFR modulator, with compounds of formula I, especially Compound A or a pharmaceutically acceptable salt thereof. Possible EGFR modulators that upon treatment can result in resistance are, for instance, gefitinib, erlotinib, lapatinib, cetuximab, nimotuzumab, panitumumab, and trastuzumab.

A compound of the formula (I) may also be used for the treatment of EGFR dependent or EGFR acquired resistance diseases in combination with other active compounds for instance the combination partners as disclosed in WO07/084786, more preferred EGFR family targeting agents such as, and without limitation to gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922

The present disclosure also describes a combination treatment of EGFR dependent diseases with a compounds of formula I, especially of a compound selected from the group consisting of (Compound A) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and/or ovarian cancer

In particular, the present disclosure describes a combination of compound of formula I selected from the group consisting of 5-(2,6-di-morpholin-4-yl-pyrimidin-4-yl)-4-trifluoromethyl-pyridin-2-ylamine and an EGFR modulator selected from the group consisting of gefitinib, erlotinib, lapatinib, cetuximab, nimotuzumab, panitumumab, and trastuzumab, wherein the active ingredients are present in each case in free form or in the form of a pharmaceutically acceptable salt, and optionally at least one pharmaceutically acceptable carrier; for simultaneous, separate or sequential use for the treatment of non small cell lung carcinoma, head and neck cancer, colorectal carcinoma, breast cancer, brain malignancies including glioblastoma, prostate cancer, bladder cancer, renal cell carcinoma, pancreas cancer, cervical cancer, esophageal cancer, gastric cancer and ovarian cancer.

In another embodiment the present invention relates to a pharmaceutical preparation for use in the treatment of an EGFR dependent disease that has acquired resistance during treatment with an EGFR modulator comprising 5-(2,6-di-morpholin-4-yl-pyrimidin-4-yl)-4-trifluoromethyl-pyridin-2-ylamine (Compound A) or a pharmaceutically acceptable salt thereof and at least one pharmaceutically acceptable carrier, wherein the EGFR modulator is selected from the group consisting of gefitinib, erlotinib, lapatinib, cetuximab, nimotuzumab, panitumumab and trastuzumab and wherein the disease to be treated is non small cell lung carcinoma.

The diseases to be treated by this pharmaceutical preparation is non small cell lung carcinoma.

The present disclosure also describes the use of a compound of formula I, especially preferred 5-(2,6-di-morpholin-4-yl-pyrimidin-4-yl)-4-trifluoromethyl-pyridin-2-y lamine (Compound A) or a pharmaceutically acceptable salt thereof for the treatment of an EGFR dependent disease or disease that has acquired resistance during treatment with an EGFR modulator.

The diseases to be treated by this compound, alone or in combination with an EGFR modulator, is preferentially non small cell lung carcinoma.

A compound of the formula (I) may also be used to advantage in combination with known therapeutic processes, for example, hormone therapy or, especially, radiation. A compound of formula (I) may in particular be used as a radiosensitizer, especially for the treatment of tumors which exhibit poor sensitivity to radiotherapy.

Treatment in accordance with the invention may be symptomatic or prophylactic.

By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative, e.g. synergistic effect.

A compound of formula I can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The compounds described herein may be administered by any conventional route, in particular parenterally, for example in the form of injectable solutions or suspensions, enterally, e.g. orally, for example In the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising a compound of the disclosure in association with at least one pharmaceutical acceptable carrier or diluent may be manufactured in conventional manner by mixing with a pharmaceutically acceptable carrier or diluent.

The pharmaceutical compositions are comprising an amount effective in the treatment of one of the above-mentioned disorders, of a compound of formula I or an N-oxide or a tautomer thereof together with pharmaceutically acceptable carriers that are suitable for topical, enteral, for example oral or rectal, or parenteral administration and that may be inorganic or organic, solid or liquid. There are pharmaceutical compositions used for oral administration especially tablets or gelatin capsules that comprise the active ingredient together with diluents, for example lactose, dextrose, manitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present disclosure in the form of parenterally administrable compositions or in the form of infusion solutions. The pharmaceutical compositions may be sterilized and/or may comprise excipients, for example preservatives, stabilizers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectioning, dissolving or lyophilising lyophilizing processes, and comprise approximately from 1% to 99%, especially from approximately 1% to approximately 20%, active ingredient(s).

## Claims

1. Use of 5-(2,6-di-morpholin-4-yl-pyrimidin-4-yl)-4-trifluoromethyl-pyridin-2-ylamine, or a pharmaceutically acceptable salt thereof, for the manufacture of a pharmaceutical preparation for the treatment of an EGFR dependent disease, wherein said disease is resistant to the treatment with an EGFR modulator which is selected from the group consisting of gefitinib, erlotinib, lapatinib, cetuximab, nimotuzumab, panitumumab and trastuzumab, and wherein the EGFR dependent disease is non small cell lung carcinoma.

2. The use according to claim 1, wherein the disease is a malignancy.

3. The use according to claim1 or 2, wherein resistance to the treatment with the EGFR modulator has been acquired during treatment with said EGFR modulator.

4. 5-(2,6-di-morpholin-4-yl-pyrimidin-4-yl)-4-trifluoromethyl-pyridin-2-ylamine, or a pharmaceutically acceptable salt thereof, for use in the treatment of an EGFR dependent disease that has acquired resistance during treatment with an EGFR modulator which is selected from the group consisting of gefitinib, erlotinib, lapatinib, cetuximab, nimotuzumab, panitumumab and trastuzumab, wherein the disease to be treated is non small cell lung carcinoma.

5. A compound for use according to claim 4, wherein the disease is a malignancy.

6. A compound for use according to claim 4 or 5, wherein resistance to the treatment with the EGFR modulator has been acquired during treatment with said EGFR modulator.

7. A pharmaceutical preparation for use in the treatment of an EGFR dependent disease that has acquired resistance during treatment with an EGFR modulator selected from the group consisting of gefitinib, erlotinib, lapatinib, cetuximab, nimotuzumab, panitumumab and trastuzumab, wherein the disease to be treated is non small cell lung carcinoma, comprising 5-(2,6-di-morpholin-4-yl-pyrimidin-4-yl)-4-trifluoromethyl-pyridin-2-ylamine, or a pharmaceutically acceptable salt thereof, and at least one pharmaceutically acceptable carrier.

## Patentansprüche

1. Verwendung von 5-(2,6-Di-morpholin-4-yl-pyrimidin-4-yl)-4-trifluormethyl-pyridin-2-ylamin oder eines pharmazeutisch verträglichen Salzes davon bei der Herstellung einer pharmazeutischen Zubereitung zur Behandlung einer EGFR-abhängigen Krankheit, wobei diese Krankheit gegen die Behandlung mit einem EGFR-Modulator resistent ist, der ausgewählt ist aus der Gruppe bestehend aus Gefitinib, Erlotinib, Lapatinib, Cetuximab, Nimotuzumab, Panitumumab und Trastuzumab, und wobei die EGFR-abhängige Krankheit nicht-kleinzelliges Lungenkarzinom ist.

2. Die Verwendung nach Anspruch 1, wobei die Krankheit ein Malignom ist.

3. Die Verwendung nach Anspruch 1 oder 2, wobei die Resistenz gegen die Behandlung mit dem EGFR-Modulator während der Behandlung mit diesem EGFR-Modulator erworben wurde.

4. 5-(2,6-Di-morpholin-4-yl-pyrimidin-4-yl)-4-trifluormethyl-pyridin-2-ylamin oder ein pharmazeutisch verträgliches Salz davon zur Verwendung bei der Behandlung einer EGFR-abhängigen Krankheit, die während der Behandlung mit einem EGFR-Modulator Resistenz erworben hat, der ausgewählt ist aus der Gruppe bestehend aus Gefitinib, Erlotinib, Lapatinib, Cetuximab, Nimotuzumab, Panitumumab und Trastuzumab, wobei die zu behandelnde Krankheit nicht-kleinzelliges Lungenkarzinom ist.

5. Eine Verbindung zur Verwendung nach Anspruch 4, wobei die Krankheit ein Malignom ist.

6. Eine Verbindung zur Verwendung nach Anspruch 4 oder 5, wobei die Resistenz gegen die Behandlung mit dem EGFR-Modulator während der Behandlung mit diesem EGFR-Modulator erworben wurde.

7. Eine pharmazeutische Zubereitung zur Verwendung bei der Behandlung einer EGFR-abhängigen Krankheit, die Resistenz während der Behandlung mit einem EGFR-Modulator erworben hat, der ausgewählt ist aus der Gruppe bestehend aus Gefitinib, Erlotinib, Lapatinib, Cetuximab, Nimotuzumab, Panitumumab und Trastuzumab, wobei die zu behandelnde Krankheit nicht-kleinzelliges Lungenkarzinom ist, umfassend 5-(2,6-Di-morpholin-4-yl-pyrimidin-4-yl)-4-trifluormethyl-pyridin-2-ylamin oder ein pharmazeutisch verträgliches Salz davon und zumindest einen pharmazeutisch verträglichen Träger.

## Revendications

1. Utilisation de 5-(2,6-di-morpholin-4-yl-pyrimidin-4-yl)-4-trifluorométhyl-pyridin-2-ylamine, ou d'un sel pharmaceutiquement acceptable de celle-ci, pour la fabrication d'une préparation pharmaceutique pour le traitement d'une maladie dépendante d'EGFR, ladite maladie étant résistante au traitement par un modulateur d'EGFR qui est choisi dans le groupe consistant en géfitinib, erlotinib, lapatinib, cétuximab, nimotuzumab, panitumumab et trastuzumab, et la maladie dépendante d'EGFR étant un carcinome du poumon non à petites cellules.

2. Utilisation selon la revendication 1, dans laquelle la maladie est une malignité.

3. Utilisation selon l'une des revendications 1 ou 2, dans laquelle la résistance au traitement par le modulateur d'EGFR a été acquise pendant le traitement par ledit modulateur d'EGFR.

4. 5-(2,6-Di-morpholin-4-yl-pyrimidin-4-yl)-4-trifluorométhyl-pyridin-2-ylamine, ou sel pharmaceutiquement acceptable de celle-ci, pour l'utilisation dans le traitement d'une maladie dépendante d'EGFR qui a acquis une résistance pendant le traitement par un modulateur d'EGFR qui est choisi dans le groupe consistant en géfitinib, erlotinib, lapatinib, cétuximab, nimotuzumab, panitumumab et trastuzumab, la maladie à traiter étant un carcinome du poumon non à petites cellules.

5. Composé pour l'utilisation selon la revendication 4, dans lequel la maladie est une malignité.

6. Composé pour l'utilisation selon l'une des revendications 4 ou 5, dans lequel la résistance au traitement par le modulateur d'EGFR a été acquise pendant le traitement par ledit modulateur d'EGFR.

7. Préparation pharmaceutique pour l'utilisation dans le traitement d'une maladie dépendante d'EGFR qui a acquis une résistance pendant le traitement par un modulateur d'EGFR choisi dans le groupe consistant en géfitinib, erlotinib, lapatinib, cétuximab, nimotuzumab, panitumumab et trastuzumab, la maladie à traiter étant un carcinome du poumon non à petites cellules, comprenant la 5-(2,6-di-morpholin-4-yl-pyrimidin-4-yl)-4-trifluorométhyl-pyridin-2-ylamine ou un sel pharmaceutiquement acceptable de celle-ci, et au moins un support pharmaceutiquement acceptable.
